# EUROPEAN PATENT APPLICATION

(11) **EP 3 730 213 A1**
(43) Date of publication of application: **28.10.2020**
(21) Application number: 19170963.3
(22) Date of filing: 24.04.2019
(51) Int. Cl.: B01L 3/00, G01N 33/49, G01N 15/14, G01N 1/40

(54) **METHOD AND SYSTEM FOR PACKED BED CELL ACOUSTIC SEPARATION**

(71) Applicant: AcouSort AB, 223 81 Lund (SE)
(72) Inventor: AUGUSTSSON, Per, 417 49 GÖTEBORG (SE)
(74) Representative: Brann AB

(57) **Abstract**

A method of performing a separation of a sample of a disperse fluid comprises the steps of: i. providing a sample of a disperse fluid comprising particles dispersed in a fluid, wherein the particles comprises at least a first type of particle and at least a second type of particles, wherein the absolute value of the acoustic contrast of the first type of particle, relative to the fluid, is lower than the absolute value of the acoustic contrast of the second type of particle relative to the fluid, ii. positioning the sample in a microfluidic cavity, iii. subjecting the sample, in the microfluidic cavity, to an acoustic standing wave configured for causing the first and second type of particle to congregate in at least one first region of the cavity, thereby causing the fluid to occupy at least one second region of the cavity, and thereby defining at least one interface between the first region and the second region, and iv. collecting at least a portion of the first region adjacent and along the at least one interface to obtain the first type of particles. A system is also disclosed.

## Description

### Technical field

The technology proposed herein relates generally to the field of acoustofluidic devices, systems and methods of handling or separating particles or cells in such devices and systems. More particularly the technology proposed herein concerns using the buoyancy of a first type of cell or particle when dispersed in an acoustically congregated phase of a second type of cell or particle to separate the first type of a cell or particle from the second type of particle.

### Background

Acoustofluidic devices include microfluidic acoustofluidic chips comprising acoustofluidic, e.g. microfluidic, cavities such as channels in which a sample fluid can be processed by performing an acoustofluidic operation on the sample fluid. During the acoustofluidic operation ultrasound energy is supplied to the acoustofluidic device including any microfluidic channel or cavity to affect the sample fluid and any particles suspended in the sample fluid. The acoustofluidic operation may be one of separation, i.e. affecting movement of the particles in the sample fluid so that different particles travel different distances in the fluid as a function of how each particles is affected by the ultrasound energy being supplied to the acoustofluidic device. By suitable design of the microfluidic channels or cavities, together with suitable selection of laminar flow of the sample or no-flow conditions, different particles can be moved to different positions and thereafter separated from each other. Other acoustofluidic operations involve driving particles in the sample fluid towards a position, such as the center or walls, of the microfluidic channels or cavities.

In addition to requiring a suitable design of the of the microfluidic channel or cavity, which design may need to be complex and thus costly to manufacture, a further prerequisite for a successful separation is generally that the sample is sufficiently dilute, i.e. that the concentration of cells or particles is sufficiently low, so that each type of cell or particle is allowed to move freely to the position dictated by the acoustic field caused by the ultrasound energy, without being hindered by other particles. Ideally the cells or particles should proceed in single file through the region of the acoustic field so that the movement of each specific cell or particle is not impeded by any other cell or particle.

This however reduces the throughput of the separation. Thus time must be spent on suitably diluting each sample before it can be separated. Moreover, dilution leads to longer processing times because cells or particles become suspended in larger volumes. The low throughput prevents the application of acoustofluidic separation to large volume applications such as separation of larger volumes of blood etc. In addition, in many biomedical analytical procedures it is not acceptable to dilute the blood by additional fluids that can affect the outcome of the analysis.

Blood is an important source of information about our health. Blood tests, such as hematocrit and hemoglobin levels, white blood cell counts, blood gas, and infection parameters are routinely guiding healthcare professionals in treatment decisions. To meet demands for shorter time from sampling to result there is an increased interest to shift from analysis in centralized labs to point-of-care tests and patient self-testing for increasingly more advanced tests. Point-of-care equipment must be autonomous and robust to enable measurements by non-specialist operators, and achieve this for a reasonable price and size of equipment. Therefore, microscale technologies are sought for that can take in small amounts of blood and output results within minutes. The high precision and flexibility for multi-stage serial processing offered in microfluidic systems opens up for fast and automated isolation of rare cell populations, such as white blood cell (WBC) sub populations, circulating tumor cells, and controlled high-throughput size fractionation of bio-nanoparticles, such as pathogens and extracellular vesicles. Blood is, however, an extremely diverse and crowded suspension of cells and particles and about 40% of the blood volume consists of red blood cells (RBC) making it time consuming to isolate rare species from blood by traditional methods.

These traditional methods to fractionate blood rely on selective lysis, centrifugation and molecular labels and typically cells are exposed to series of lengthy procedures such as density gradient centrifugation (45-60 min), repeated washing (30 min), incubation with labels (30 min), repeated washing and resuspension (30 min), FACS sorting (15-360 min) etc. adding up to several hours. Shortening times of centrifugation by using higher G-forces is often not an option as centrifugation can impair cell function. In addition the cell losses in centrifugation are operator dependent. Details such as how close to the cell pellet that the operator aspirates off the supernatant, to what degree the pellet has been compacted, and the shear velocity of pipetting during re-suspension play an important role. For further purification of sub-micron biological particles such as extracellular vesicles the most common methods is ultracentrifugation, which takes several hours per round. There is accordingly a need for new separation methods that allow separation of cells or particles at high concentrations.

### Objects

Accordingly, it is an object of the technology proposed herein to provide a method of separating cells or particles that allows the concentration of cells or particles in the sample to be increased.

A further object of the technology proposed herein is to provide a method of separating whole blood into at least red blood cells and white blood cells.

### Summary

At least one of the abovementioned objects, or at least one of the further objects that will become evident from the below description, are according to a first aspect of the technology proposed herein obtained by a method of performing a separation of a sample of a disperse fluid comprising the steps of:
i. providing a sample of a disperse fluid comprising particles dispersed in a fluid, wherein the particles comprises at least a first type of particle and at least a second type of particles, wherein the absolute value of the acoustic contrast of the first type of particle, relative to the fluid, is lower than the absolute value of the acoustic contrast of the second type of particle relative to the fluid,
ii. positioning the sample in a microfluidic cavity,
iii. subjecting the sample, in the microfluidic cavity, to an acoustic standing wave configured for causing the first and second type of particle to congregate in at least one first region of the cavity, thereby causing the fluid to occupy at least one second region of the cavity, and thereby defining at least one interface between the first region and the second region, and
iv. collecting at least a portion of the first region adjacent and along the at least one interface to obtain the first type of particles.

Accordingly the technology proposed herein is based on the discovery that, when particles are packed together towards an acoustic pressure node of an acoustic field/acoustic standing wave, the particles having the lower absolute value of acoustic contrast relative to the fluid, which for particles having a positive acoustic contrast relative to the fluid are the particles that are less dense or more compressible particles (first type of particles), will be displaced away from the pressure node by the particles having the higher acoustic contrast, in this case more dense or less compressible particles (second type of particles), due to the interparticular forces, i.e. the forces or pressure between the particles, which result in a net force away from the pressure node. The particles with lower acoustic contrast relative to the fluid may thus be considered to experience a force similar to buoyancy whereby these particles rise up through the particles with higher acoustic contrast relative to the fluid towards the interface between the packed particles and the suspending fluid. The result is that the particles with lower acoustic contrast relative to the fluid congregate at the interface between the packed particles and the suspending fluid. In addition the initial movement of the particles towards the pressure node gives rise to a counter flow of suspending fluid, which counter flow flush out smaller particles or species that the disperse fluid may contain towards the second region.

The first and second type particle preferably either both have a positive acoustic contrast relative to the fluid or alternatively a negative acoustic contrast relative to the fluid to cause them to congregate in the at least one first region.

If the first and second type particle both have a positive acoustic contrast relative to the fluid, then the first and second type of particle will initially generally congregate towards the pressure node. This is typically the center region of the microfluidic cavity or, if the acoustic standing wave comprises several pressure nodes, in several generally centrally located first regions.

If, on the other hand, the first and second type particle both have a negative acoustic contrast relative to the fluid, then the first and second type of particle will initially generally congregate away from the pressure node, i.e. towards an antinode. This is typically two peripherally located first regions of the microfluidic cavity or, if the acoustic standing wave comprises several pressure anti-nodes, in several first regions.

In both cases the first type of particle, due to its lower absolute value of its acoustic contrast relative to the fluid, will eventually be congregated along the at least one interface.

The acoustic contrast is dependent on density and compressibility. Thus the first type of particle may have either a lower density, or a higher compressibility, or both, relative to the second type of particle when the acoustic contrast of both the first and second types of particle is positive relative to the fluid, or vice versa.

This effect may be used either for separating the first type of particle from the second type of particle, or vice versa.
Thus step iv provides for obtaining the first type of particles. Step iv further provides for obtaining the second type of particles as the remainder of the first region after the portion has been collected.
In step iv at least a portion of the first region adjacent and along the interface is collected to obtain the first type of particles. Typically, in addition to the portion, parts of the second region, i.e. parts of the suspending fluid, are also collected in step iv.

Where it is desired to obtain the first type of particle only, then preferably only the portion is collected. On the other hand, if it is desired to obtain the second type of particle only, then step iv preferably comprises collecting the portion of the first region and also the suspending fluid in the second region or regions.

It is further contemplated within the context of the technology proposed herein that step iv may be dispensed with or replaced with a step of determining a property of the sample, the first type of particles or the second type of particles without collecting the portion. This may for example be the case where the separation of the particles into the first region with the first type of particles being present in a portion of the first region adjacent and along the interface provides a useful starting point for analysis. It may for example be of interest to determine a property of the second type of particle. A measurement in the first region at a position away from the portion, (i.e. at the maximum distance from the interface) ensures that no particle of the second type of particle is present where the measurement is taken, thus increasing accuracy. In a further example it may be of interest to determine the proportion of first type of particles to second type of particles. Such information may be obtained by measuring the presence of the first and second type of particles, for example optically (by fluorescence), and determining the amount of the first type of particles along the interface in relation to the amount of the second type of particles in the remainder of the first region.
Accordingly step iv may be replaced by a step v of performing a measurement in or of the portion and/or the remainder of the first region, and/or determining the amount of the first type of particle and/or the amount of the second type of particles in the first region.

The sample should be in fluid form and in a viscosity suitable for it being positioned in the cavity.

The disperse fluid may for example comprise undiluted or diluted whole blood, interstitial fluid, synovial fluid, peritoneal fluid, urine, yeast cell cultures, bone marrow, stroma, dissociated cells from normal or cancerous tissue, milk.
The particles may comprise red blood cells, white blood cells, platelets, cancer cells, circulating tumor cells, bacterial cells, viruses, yeast cells, fat cells, exosomes, extracellular vesicles, microvesicles, lipoproteins, dust particles, silica particles and polymer particles.

The fluid may be plasma, water, urine, yeast cell broth, cell culture medium, saline solutions, phosphate buffered saline, interstitial fluid, milk plasma. The disperse fluid is preferably whole blood whereby the first type of particle is white blood cells or leukocytes and the second type of particle is red blood cells or erythrocytes, and the fluid is blood plasma.

The disperse fluid may comprise further types of particles having an acoustic contrast, the absolute values of which are between those of the first and second types of particles.

The sample may be positioned in the cavity by pumping, by pressure, by suction, by the action of electrical fields, by gravity, and/or by capillary action.

The microfluidic cavity may be closed to the environment. The microfluidic cavity is preferably a channel having a square or rectangular cross section. The microfluidic cavity may for example have a cross sectional width of 1 to 10 times, such as 1 to 2 times the cross sectional height. In this case the length of the microfluidic cavity is at least the same as the width, but preferably many times longer. The width may for example be from 0.15 mm to 1 mm. The height may for example be from 0.1 mm to 5 mm. Generally, to scale up the method and system the height and/or width may be further increased. Preferably the dimension in which the standing wave is directed (typically the width) may be kept constant and the other dimension (in this case the height) increased. It is for example possible to use a cavity having a height several times larger, such as 10, 20, 50 or 100 times larger than the width.

The microfluidic cavity has at least one resonance frequency. Resonance frequencies of the microfluidic cavity are dependent on the dimensions because, in order for a standing wave to form the wavelength λ of the wave, which wavelength is inversely proportional to the frequency, must be nλ/2 where n is a positive integer. The sample may be subjected to the acoustic standing wave by an ultrasound transducer attached to the microfluidic cavity or to the substrate.

The following are the first 3 resonances for a standing wave directed along the width dimension of the cavity for particles with positive acoustic contrast relative to the suspending fluid:
a first resonance frequency **f** is associated with an acoustic standing wave λ/2 corresponding to a first harmonics w here the pressure anti nodes of the standing wave are positioned near the walls of the cavity and a single pressure node is formed in the middle of the cavity, thus causing the particles to congregate in the center of the cavity, this being the first region and the regions near the side walls of the cavity being the second regions. Thus we get: wall 1 - fluid 1 - particles 1 - fluid 2-wall 2. This resonance frequency provides two interfaces adjacent and along which the first type of particle can be collected. This is the preferred resonance frequency as it simplifies the collection of the portion of the first region adjacent and along an interface.

In a second harmonics, corresponding to a resonance frequency that is two times the first resonance frequency, i.e. **2f**, a standing wave λ is formed having two nodes and three antinodes, thus generally forcing the particles to congregate in two bands on both sides of the center of the cavity, this being the first regions and the center and sides of the cavity being the second regions. Thus we get: wall 1 - fluid 1 - particles 1 - fluid 2 - particles 2 - fluid 3 - wall 2.
This resonance frequency provides four interfaces adjacent and along which the first type of particle can be collected.

Accordingly the term resonance frequency is to be understood to comprise any frequency in which a standing wave may form in the cavity, and a frequency causing the formation of a standing wave is considered a frequency configured for causing the particles to congregate in at least one first region.

The at least one first region of the cavity is preferably a central region of the cavity. The resonance frequency of the cavity, and thus the frequency of the acoustic standing wave, may be from 0.15 MHz to 10MHz, preferably from 1 MHz to 10 MHz. The frequency of the acoustic standing wave may be fixed or varied around the resonance frequency.

The interface is defined between the at least one first region and the at least one second region. Where the first region is a central region, two second regions and conversely two interfaces will be defined, one on each side of the first region. Generally the portion is the part of the first region along the interface in which the first type of particles congregate.

The size and extent of the portion depends on the total concentration of particles in the sample, and the concentration of the first type of particles amount the particles. If the concentration of the first type of particles is high, then the portion will comprise more of the first region, and vice versa. The desired specificity also impacts the desired size of the portion. Thus, if a high specificity in collecting the first type of particles is desired, the size of the portion, specifically the width of the portion transverse to along the interface, should be small so as to minimize the amount of particles of the second type of particles that are collected along with the first type of particles. A small width of the portion may lead to some of the first type of particles not being collected and remaining in the remainder of the first region together with the second type of particles. A larger width of the portion allows more of the first type of particles to be collected, while increasing the risk that also particles of the second type of particles are collected. This however decreases the risk or incidence that the remainder of the first region contains any particles of the first type of particle, and may therefore be advantageous where the second type of particle is desired to be collected.

The width of a portion may be from 0.01 % to 49% of the width of the first region, such as from 1 to 40%, 2 to 40%, 1 to 20%, 2 to 20%, 1 to 10%, 2 to 10%, 1 to 5% or 2 to 5% of the width of the first region.

Although the method and system according to the first and second aspects of the technology proposed herein generally mention a first type of particle and a second type of particle, the method and system may be used with disperse fluid comprising multiple type of particles. In these cases each type of particle will be arranged in the first region by order of the absolute value of acoustic contrast relative to the fluid, with interfaces between each two types of particles differing in the absolute value of the acoustic contrast. The outermost interface will be between the first region and the second region, along which particles with the lowest absolute value of acoustic contrast relative to the fluid will be arranged. As above the portion may be selected so as to contain the particles with lower acoustic contrast relative to the fluid. Alternatively the portion may be selected so as to contain several types of particles and several interfaces between different types of particles. It may for example be desired to collect, and thereby separate, all particles that have a lower absolute value of acoustic contrast relative to the fluid than a type of particle having the highest absolute value of acoustic contrast relative to the fluid. It may also be desirable to collect a portion containing different types of particles, which have a lower absolute value of acoustic contrast relative to the fluid than other particles, in order to further separate the particles in the collected portion by running the collected portion through the acoustic standing wave again, whereby the particles having the highest absolute value of acoustic contrast relative to the fluid will now be found closest to the pressure node (for positive acoustic contrast) or closest to the antinode (negative acoustic contrast). It may for example be useful to provide two or more microfluidic cavities in series, wherein the collected portion from an upstream cavity, or alternatively the remainder of the first region from said upstream cavity, is further separated in a downstream cavity subjecting it to an acoustic standing wave and by collecting a second portion of the first region forming in said downstream cavity.

Where the sample flows through the first cavity the size and dimension of the portion may be determined by the flow rate of sample flowing into the cavity, the flow rate of sample flowing out of the cavity from along the interface, and the flow rate of sample flowing out of the cavity from the remainder of the first region. If for example the total volume fraction of particles in the disperse fluid is 50%, then the packed volume of the particles will occupy about 50% of the total volume (typically the packed volume of the particles will occupy a somewhat larger volume than 50% of the total volume because of some suspending fluid remaining between the particles). If the 50% of the particles are of the first type and 50% are of the second type, then the first type particles will then, after being focused, occupy slightly more (due to the remaining fluid) than 25% of the channel (cavity) cross section. If the cavity has a central outlet, and if the acoustic contrast of the first and second type of particle is positive relative to the fluid, then setting the flow rate through this outlet to 25% of the total flow allows the second type of particles to be recovered through that outlet, whereas the portion comprising the first type of particle is collected using side outlets set to the inlet flow rate minus the central outlet flow rate. The relative flow rate of the central outlet flow can be increased to increase the recovery of the second type of particles, or the flow can be decreased to increase the purity of the second type of particles, or vice versa.
For blood the packed blood cell volume (hematocrit) is typically 45% but it can vary from 25% to 60%. The white blood cells normally constitute less than 1% of the total cell volume of a sample. White blood cells would thus only be present in a thin layer at the interface between the red blood cells and the plasma. The blood cells have a positive acoustic contrast relative to the plasma and here the central outlet flow rate should be set to a value close to the hematocrit (such as percentage of 1/25 to 1/60 below the hematocrit). The central outlet flow rate may then be adjusted while viewing the cavity using a microscope so that the majority of the red blood cells are recovered through a central outlet and the white blood cells are recovered together with the plasma through the side outlet.

The portion may be collected by providing an outlet in fluid connection with the cavity, the outlet extending into the microfluidic cavity to establish a fluid connection with the portion.

Collecting the at least one portion of the first region adjacent and along the interface to obtain the first type of particles encompasses selectively collecting the at least one portion of the first region adjacent and along the interface to obtain the first type of particles. As used herein, selectively encompasses that that less than 30 wt%, preferably less than 20 wt%, more preferably less than 10 wt%, even more preferably less than 5 wt% of the portion is made up of particles of the second type of particles.

Alternatively the cavity may comprise a branching point where different spatial parts of the contents of the cavity are separated into different secondary cavities or channels, and the portion is separated into one secondary cavity or channel that is separate from the secondary cavity or channel into which the remainder of the first region enters.

Preferably the portion may be collected by providing a first outlet in fluid connection with the cavity and arranged in correspondence with the pressure node (if the particles have a positive contrast relative to the fluid), or the antinode (if the particles have a positive contrast relative to the fluid) of the acoustic standing wave to thereby collect the second type of particles, and by providing at least one second outlet arranged at the wall of the cavity away from the pressure node (when the particles have a positive contrast relative to the fluid), and by controlling the flow through the first and second outlets so that the portion and the suspending fluid in the second region is collected through the second outlet, and so that the remainder of the first region is collected through the first outlet.

In one embodiment of the method according to the first aspect of the technology proposed herein the acoustic contrast of the particles is positive relative to the fluid and the acoustic standing wave is configured for causing the particles to congregate in one central first region of the microfluidic cavity. This is advantageous as it allows the remainder of the first region to be collected using a central outlet, and the portion to be collected through one or two side outlets.

In an alternative embodiment of the method according to the first aspect of the technology proposed herein the acoustic standing wave, and/or the cavity, is configured for causing the particles to congregate in more than two first regions of the cavity. This may be advantageous as it provides additional interfaces adjacent to and along which to collect the portion of the first regions. This increases the throughput of the method if the concentration of the first type of particles is large.

In another embodiment of the method according to the first aspect of the technology proposed herein the acoustic contrast of the particles is negative relative to the fluid and the acoustic standing wave is configured for causing the particles to congregate in two peripherally located first regions of the microfluidic cavity. Preferably the two peripherally located first regions are located along the sidewalls of the microfluidic cavity. This allows the second type of particle to be collected by one, or preferably two, side outlets in the walls of the microfluidic cavity.

In the preferred embodiment of the method according to the first aspect of the technology proposed herein the method further comprises the step of d) generating a flow of the sample through the cavity. This is advantageous as it allows the method to be used for inline and online optical or electrical measurements.

The flow may be generated by pumping, by pressure, by suction, by the action of electrical fields, by gravity, and by capillary action.

The cavity may be fluidly connected to an inlet, through which the sample is introduced into the cavity, and fluidly connected to an outlet, through which the sample leaves the cavity. More than one outlet may be fluidly connected to the cavity in order to lead off different parts of the sample to different outlets.

In the preferred embodiment of the method according to the first aspect of the technology proposed herein the cavity is elongated and fluidly connected at one end to an inlet and at another opposite end to at least one outlet. This is advantageous as it allows the use of simple glass capillaries for providing the cavity. Typically the length of the cavity should be at least 5 times its width. In some embodiments the transverse dimension of the cavity is larger than the transverse dimensions of an inlet and an outlet to the cavity.

In the preferred embodiment of the method according to the first aspect of the technology proposed herein the cavity is formed in a substrate. The substrate may be made of silicon but may be made of polymeric material such as plastic, or alternatively glass. Also other materials such as ceramics and metals are possible. These materials are cheap and therefore suitable for performing the optical or electrical measurements in the field on in a point of care setting, as disposable consumables.

The substrate may be planar, such as a chip, or alternatively the substrate may be formed as a capillary.

In the preferred embodiment of the method according to the first aspect of the technology proposed herein ultrasound energy, for causing the acoustic standing wave is transferred to the substrate from at least one ultrasound transducer connected to the substrate.

The ultrasound transducer may be connected to the substrate by adhesives, or by being positioned so that they contact each other.

In the preferred embodiment of the method according to the first aspect of the technology proposed herein the sample is a blood sample whereby the particles comprises at least cells and the fluid comprises at least blood plasma. The cells may be mammalian cells in the circulation, such as blood cells or circulating tumor cells. The first type of particles may for example be white blood cells or circulating tumor cells, while the second type of particles may be red blood cells.

It is expected that flow rates of up to 100 µL/min whole blood or more can be separated.

At least one of the abovementioned objects, or at least one of the further objects which will become evident from the below description, are according to a second aspect, corresponding to the first aspect, of the technology proposed herein achieved by a microfluidic system for performing a separation of a sample of a disperse fluid, the disperse fluid comprising particles dispersed in a fluid and the particles comprising at least a first type of particle and at least a second type of particles, wherein the absolute value of the acoustic contrast of the first type of particle, relative to the fluid, is lower than the absolute value of the acoustic contrast of the second type of particle relative to the fluid, the system comprising:
a substrate with a microfluidic cavity formed in the substrate, the microfluidic cavity having an inlet for allowing the sample into the microfluidic cavity,
an ultrasound transducer connected to the substrate for generating an acoustic standing wave in the microfluidic cavity,
a drive circuit connected to the ultrasound transducer and configured to drive the ultrasound transducer with a frequency configured to cause the particles to congregate in at least one first region of the cavity, thereby causing the fluid to occupy at least one second region of the cavity and thereby defining at least one interface between the first region and the second region, and
a collecting device arranged and configured to collect at least a portion of the first region adjacent and along the at least one interface to obtain the first type of particles.

As discussed above for the method the collecting device may comprise an outlet in fluid connection with the cavity, the outlet extending into the microfluidic cavity to establish a fluid connection with the portion.

Alternatively the collecting device may comprise a branching point where different spatial parts of the contents of the cavity are separated into different secondary cavities or channels, and the portion is separated into one secondary cavity or channel that is separate from the secondary cavity or channel into which the remainder of the first region enters.

Alternatively or additionally pumps may be used to determine the portion by determining the flow rate of sample flowing into the cavity, the flow rate of sample flowing out of the cavity from along the interface, and the flow rate of sample flowing out of the cavity from the remainder of the first region. If for example the total volume fraction of particles in the disperse fluid is 50%, then the packed volume of the particles will occupy about 50% of the total volume (typically he packed volume of the particles will occupy a somewhat larger volume than 50% of the total volume because some suspending fluid remaining between the particles). If the 50% of the particles are of the first type and 50% are of the second type, then the first type particles will then, after being focused, occupy slightly more (due to the remaining fluid) than 25% of the channel (cavity) cross section. If the acoustic contrast of the particles is positive and the cavity has a central outlet then setting the flow rate through this outlet to 25% of the total flow allows the second type of particles to be recovered through that outlet, whereas the portion comprising the first type of particle is collected using side outlets set to the inlet flow rate minus the central outlet flow rate.

The relative flow rate of the central outlet flow can be increased to increase the recovery of the second type of particles, or the flow can be decreased to increase the purity of the second type of particles, or vice versa.

The collecting device may be arranged and configured to selectively collect at least a portion of the first region adjacent and along the interface to obtain the first type of particles.

As discussed above for the method according to the first aspect of the technology proposed herein, the first type of particle will be enriched along the interface between the first and second region. From here it can be collected by the collecting device.

The microfluidic system may further comprise a container for storing the sample, a pump or other device for causing the sample to enter the cavity, a temperature control device for heating or cooling the substrate as needed, and receptacles for receiving the portion and/or the first region
As above the substrate may be made of silicon, polymers such as plastic, or glass.
As above the microfluidic cavity is preferably elongated.

The ultrasound transducer is preferably a piezoelectric actuator. The ultrasound transducer may be connected to the substrate. The acoustic standing wave is generated by the vibrations in the ultrasound transducer being transferred to the substrate and causing the walls of the cavity to vibrate.

The drive circuit may comprise a function generator electrically connected to the ultrasound transducer. The drive circuit is configured, by comprising a function generator, to drive the ultrasound transducer with a frequency that varies. As above the frequency may be varied in several ways.

To provide a sufficiently strong acoustic field, the signal to the transducer can be amplified by an amplification circuit.

In order to monitor and reproducibly and adaptively tune the relative flow rates in the inlets and outlets in the system a camera microscope can be arranged so that it can monitor the outlet of the microfluidic cavity. The interface between the first and second regions can then be identified by analyzing the light intensity in the captured images by image analysis. The position of the interface, and thus the portion can be regulated by adjusting the relative flow rates in the outlets by for example adjusting the pressures in outlet containers into which the outlets discharge into.

In order for the ultrasound transducer to be able to generate the acoustic standing wave in the cavity it must connect to the substrate. This connection may be established by physical contact.

In some embodiments of the system according to the second aspect of the technology proposed herein the cavity is elongated and fluidly connected at one end to an inlet and at another opposite end to an outlet, as described above.

Further advantages with and features of the technology proposed herein will be apparent from the other dependent claims as well as from the following detailed description of preferred embodiments.

### Brief description of the drawings and detailed description

A more complete understanding of the abovementioned and other features and advantages of the technology proposed herein will be apparent from the following detailed description of preferred embodiments in conjunction with the appended drawings, wherein:
- Fig. 1A-B: are bright field and corresponding fluorescence microscopy images of a sample of whole blood in stopped flow being subjected to an acoustic standing wave in a cavity. The red blood cells are shown packed into a first region flanked by second regions of blood plasma, and cancer cells of the DU 145 prostate cancer cell lines are revealed adjacent and along the interface of the first and second regions due to their capability of fluorescing,
- Fig. 1C and D: are bright field and corresponding fluorescence microscopy images of a sample of whole blood being subjected to an acoustic standing wave in a cavity under the same conditions as in figs. A and B, but with the sample flowing through the cavity.
- Fig. 2: is a schematic image of the forces affecting packed red blood cells and white blood cells in the packed red blood cells,
- Fig. 3A: is a schematic view from above of a substrate and microfluidic cavity of a system according to the second aspect of the technology proposed herein, and
- Fig. 3B: is a schematic view from above of a substrate and microfluidic cavity of an alternative system according to the second aspect of the technology proposed herein.

In the figures and the description the same reference numeral is used to refer to the same feature. A ' added to a reference numeral indicates that the feature so referenced has a similar function, structure or significance as the feature carrying the reference numeral without the ', however not being identical with this feature.

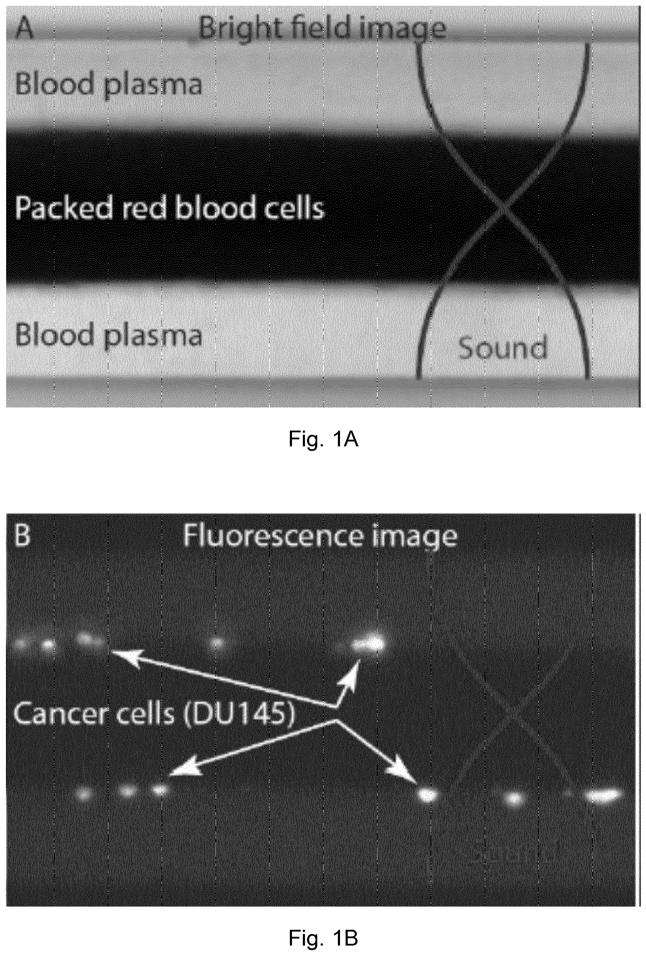

Fig. 1A-B are bright field and corresponding fluorescence microscopy images of a sample of whole blood in stopped flow being subjected to an acoustic standing wave in a cavity. The red blood cells are shown packed into a first region flanked by second regions of blood plasma, and cancer cells of the DU 145 prostate cancer cell lines are revealed adjacent and along the interface of the first and second regions due to their capability of fluorescing. The red blood cells are shown packed, i.e. congregated, into a first region flanked by second regions of blood plasma. The whole blood comprises cultured prostate cancer cells DU145 which were fluorescently labeled and spiked in the whole blood from a healthy volunteer. The blood was drawn into a syringe and then infused through a silicon and glass micro channel with a rectangular cross-section (width 375 µm, height 150 µm). After the flow had come to rest, a sound field of a frequency of 2 MHz was activated by an electrical signal submitted to a piezoelectric transducer from a signal generator and amplifier. The final stage of cell congregation was recorded by bright field and fluorescence microscopy after a few seconds of actuation. Fig. 1B, which is an overlay of 10 consecutive fluorescence images, shows that the cancer cells are congregated adjacent and along the interface of the first and second. In other words the DU145 cells have congregated at the interfaces between the packed red blood cells and the plasma. The fluorescing cancer cells have a lower absolute value of the acoustic contrast relative to the blood plasma (in this case a lower density and or higher compressibility) than the red blood cells and are therefore, when present in the almost continuous phase or environment of red blood cells, affected by buoyancy forces causing the cancer cells to rise out of the red blood cells.

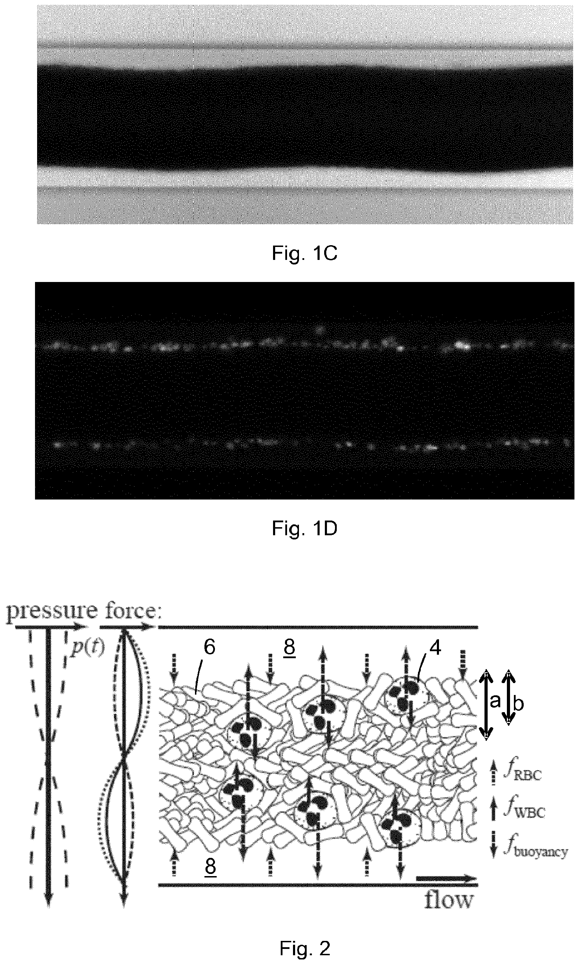

Fig. 1C and D are bright field and corresponding fluorescence microscopy images of a sample of whole blood being subjected to an acoustic standing wave in a cavity under the same conditions as in figs. A and B, but with the sample flowing through the cavity. Here the flow rate was 20 µL/min flow rate. Fig. 1D, similarly to Fig 1B, shows that the DU145 cancer cells are congregating at the cell plasma interface due to cell-cell interactions. Fig. 1A-D thus show that the method of separation

Fig. 2 is a schematic image of the forces affecting the packed red blood cells and white blood cells in the packed red blood cells. In the blood the blood cells have a positive acoustic contrast relative to the blood plasma. As shown the acoustic standing wave has a central pressure node. The red blood cells 6, which have a positive acoustic contrast in relation to the blood plasma, are forced towards the center of the cavity, see dotted arrows. This forms an almost continuous phase or packed bed of red blood cells. In this environment the white blood cells 4, which are also forced by the acoustic field towards the center due to their positive acoustic contrast in relation to he plasma, instead, due to having a lower absolute value of acoustic contrast, in relation to the plasma, than the absolute value of acoustic contrast of the red blood cells relative to the plasma, are affected by even larger net forces (dashed arrows) which causes the white blood cells to rise through the red blood cells towards the interface between the packed red blood cells 6 and the surrounding blood plasma 8. The white blood cells are therefore positioned in the portion of the first region that is adjacent and along the interface between the packed red blood cells and the blood plasma. The white blood cells can then be obtained by collecting the portion of the first region. If the objective is to collect as many white blood cells as possible, then the portion of the first region is defined larger, i.e. a larger proportion, indicated with **a** in the figure, of the width of the first region is collected. This however increases the risk that red blood cells are also collected with the first portion, i.e. this increases the quantity of the separation but reduces the quality.

If on the other hand the requirement is that as few as possible red blood cells should be collected together with the white blood cells then the portion is made smaller, i.e. a smaller proportion, indicated with **b** in the figure, of the width of the first region may be collected.

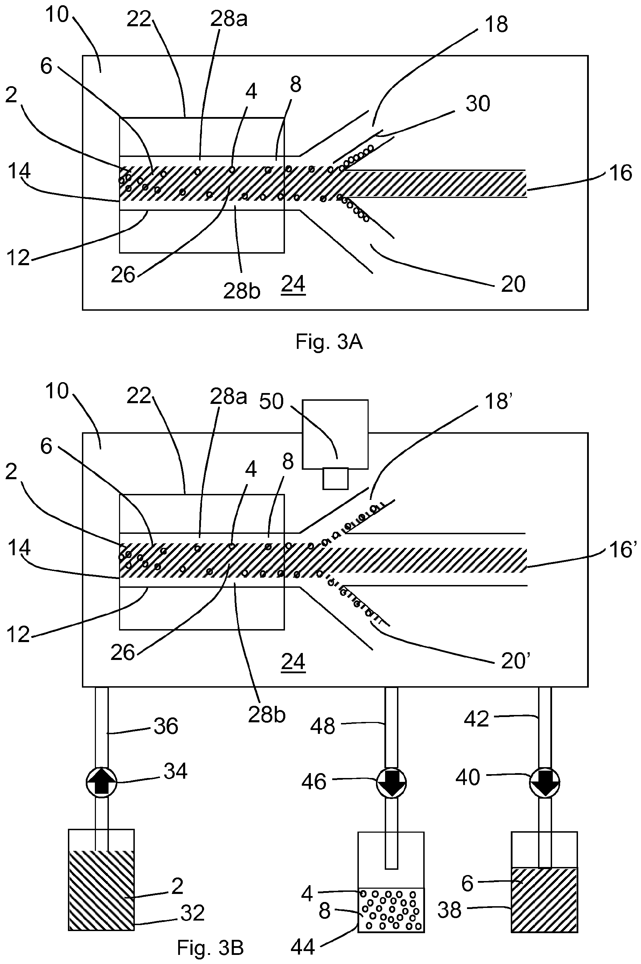

Fig. 3A is a schematic view from above of a substrate and microfluidic cavity of a system according to the second aspect of the technology proposed herein. Fig. 3 shows a silicon substrate 10 which has formed in it a microfluidic channel 12, defining a cavity, having an inlet 14, a central outlet 16, and two side outlets 18 and 20. An ultrasound transducer 22 is attached to the underside of the substrate 10, and the channel is delimited vertically by a glass sheet 24 bonded to the top of the substrate 10. The ultrasound transducer 22 may also be attached to the side of the substrate 10. A sample of whole blood 2 is admitted into the channel 12 through inlet 14 and caused to flow towards the outlets 16, 18, and 20. The sample comprises a first type of particle, i.e. white blood cells, one of which is designated the reference numeral 4, which are mixed with the red blood cells generally designated the reference numeral 6. An ultrasound transducer 22 is connected to the substrate 10 and activated with a frequency which provides an acoustic standing wave in the cavity 14. This causes the red blood cells 6 to congregate towards the central pressure node of the acoustic standing wave thus causing the red blood cells 6 to congregate in a first region, corresponding to the center portion 26, of the channel 12. At the same time the white blood cells 4, which are now dispersed in an almost continuous phase of red blood cells 6, start to rise, due to lower density and higher compressibility in relation to the red blood cells, towards the interface of the first region and the second regions 28a and 28b containing the plasma 8. The white blood cells 4 are thus concentrated towards a portion of the first regions lying adjacent and along the interface between the first and second regions 26 and 28a, 28b.

A collecting device, here implemented as the branching of the cavity into the central and side outlets 16, 18, 20, skims off the portion of the first region 26, for example the outermost 5 to 10% of the first region, and leads this portion into the side outlets 18 and 20. This causes the white blood cells 4, which were driven towards the interface of the first region 26 and the second regions 28a, 28b, to be diverted, together with the majority of the blood plasma 8 in the second regions 28a and 28b, into the side outlets 18 and 20 from which the white blood cells 4 can be obtained.

If desired further features, such as guide wall 30, may be introduced into the side outlets to reduce the amount of plasma that is collected together with the white blood cells.

Whereas some red blood cells 6 will be collected together with the white blood cells, the method is capable of collecting all of the white blood cells 4 while collecting less than 10 of the red blood cells 6. Further, the central outlet 16 provides for collecting the red blood cells 6

The portion of the first region that is collected is can be set according to the flow and volume of the sample, or alternatively a fixed geometry can be used and the flow of sample adjusted until the total width of the first region gives a suitable portion or "skimming depth".

This is inter alia shown in Fig. 3B which is a schematic view from above of a substrate and microfluidic cavity of an alternative system according to the second aspect of the technology proposed herein. Here the collecting device is represented by a branching of the cavity into the central and side outlets 16', 18', 20', which, in contrast to central and side outlets 16, 18 and 20 of Fig. 3A do not physically interfere with the flow of the sample, rather the flow rates of the inlet 14 and in the central and side outlets 16', 18' and 20' are set so as to cause the portion of the first region together with the second regions 28a, 28b containing the plasma to be led out through the side outlets 18' and 20' whereas the remainder of the first region 26 is led out through the central outlet 16'. Thus whole blood 2 is led from a container 32 via first pump 34 and tubing 36 to the inlet 14. The remainder of the first region 26 containing the red blood cells 6 is collected from the central outlet 16' into container 38 using second pump 40 and tubing 42. The portion of the first region containing the white blood cells 4 together with the second regions 28a, 28b containing the plasma 8 are collected through side outlets 18' and 20' into container 44 using third pump 46 and tubing 48. The second pump 40 is set to a flow that is approximately the same as the content of blood cells (both red and white) in the whole blood 2. A microscope of camera 50 may be placed to image the branching of the channel 12 into the central and side outlets 16', 18' and 20', and to thereby provide information on the separation for adjusting the second and third pumps 40 and 46. As an alternative the containers 32, 38 and 44 may be pressurized differently to drive the flow and set the flow rates instead of, or in addition to, using the pumps 34, 40 and 46.

### Feasible modifications

The technology proposed herein is not limited only to the embodiments described above and shown in the drawings, which primarily have an illustrative and exemplifying purpose. This patent application is intended to cover all adjustments and variants of the preferred embodiments described herein, thus the present invention is defined by the wording of the appended claims and the equivalents thereof. Thus, the equipment may be modified in all kinds of ways within the scope of the appended claims.

For instance, it shall be pointed out that structural aspects of embodiments of the method according to the first aspect of the technology proposed herein shall be considered to be applicable to embodiments of the system according to the second aspect of the technology proposed herein, and conversely, methodical aspects of embodiments of the system according to the second aspect of the technology proposed herein shall be considered to be applicable to embodiments of the method according to the first aspect of the technology proposed herein.

It shall also be pointed out that all information about/concerning terms such as above, under, upper, lower, etc., shall be interpreted/read having the equipment oriented according to the figures, having the drawings oriented such that the references can be properly read. Thus, such terms only indicates mutual relations in the shown embodiments, which relations may be changed if the inventive equipment is provided with another structure/design.

It shall also be pointed out that even thus it is not explicitly stated that features from a specific embodiment may be combined with features from another embodiment, the combination shall be considered obvious, if the combination is possible.

Throughout this specification and the claims which follows, unless the context requires otherwise, the word "comprise", and variations such as "comprises" or "comprising", will be understood to imply the inclusion of a stated integer or steps or group of integers or steps but not the exclusion of any other integer or step or group of integers or steps.

## Claims

1. A method of performing a separation of a sample of a disperse fluid comprising the steps of:
v. providing a sample of a disperse fluid comprising particles dispersed in a fluid, wherein the particles comprises at least a first type of particle and at least a second type of particles, wherein the absolute value of the acoustic contrast of the first type of particle, relative to the fluid, is lower than the absolute value of the acoustic contrast of the second type of particle relative to the fluid,
vi. positioning the sample in a microfluidic cavity,
vii. subjecting the sample, in the microfluidic cavity, to an acoustic standing wave configured for causing the first and second type of particle to congregate in at least one first region of the cavity, thereby causing the fluid to occupy at least one second region of the cavity, and thereby defining at least one interface between the first region and the second region, and
viii. collecting at least a portion of the first region adjacent and along the at least one interface to obtain the first type of particles.

2. The method according to claim 1, wherein the acoustic contrast of the particles is positive relative to the fluid and the acoustic standing wave is configured for causing the particles to congregate in one central first region of the microfluidic cavity.

3. The method according to claim 1, wherein the acoustic contrast of the particles is negative relative to the fluid and the acoustic standing wave is configured for causing the particles to congregate in two peripherally located first regions of the microfluidic cavity.

4. The method according to any preceding claim, further comprising the step of generating a flow of the sample through the microfluidic cavity.

5. The method according to claim 4, wherein the cavity is elongated and fluidly connected at one end to an inlet and at another opposite end to at least one outlet.

6. The method according to any preceding claim, wherein the cavity is formed in a substrate.

7. The method according to claim 6, wherein ultrasound energy, for causing the acoustic standing wave, is transferred to the substrate from at least one ultrasound transducer connected to the substrate.

8. The method according to any preceding claim, wherein the sample is a blood sample whereby the particles comprises at least cells and the fluid comprises at least blood plasma.

9. A microfluidic system for performing a separation of a sample of a disperse fluid, the disperse fluid comprising particles dispersed in a fluid and the particles comprising at least a first type of particle and at least a second type of particles, wherein the absolute value of the acoustic contrast of the first type of particle, relative to the fluid, is lower than the absolute value of the acoustic contrast of the second type of particle relative to the fluid, the system comprising:
a substrate with a microfluidic cavity formed in the substrate, the microfluidic cavity having an inlet for allowing the sample into the microfluidic cavity,
an ultrasound transducer connected to the substrate for generating an acoustic standing wave in the microfluidic cavity,
a drive circuit connected to the ultrasound transducer and configured to drive the ultrasound transducer with a frequency configured to cause the particles to congregate in at least one first region of the cavity, thereby causing the fluid to occupy at least one second region of the cavity and thereby defining at least one interface between the first region and the second region, and
a collecting device arranged and configured to collect at least a portion of the first region adjacent and along the at least one interface to obtain the first type of particles.
